(19) **Europäisches Patentamt**
**European Patent Office**
**Office européen des brevets**

(11) **EP 2 211 692 B1**

(12) **EUROPEAN PATENT SPECIFICATION**

(45) Date of publication and mention
of the grant of the patent:
**15.04.2015 Bulletin 2015/16**

(51) Int Cl.:
***A61B 5/00*** (2006.01)

(21) Application number: **08839915.9**

(22) Date of filing: **16.10.2008**

(86) International application number:
**PCT/IT2008/000649**

(87) International publication number:
**WO 2009/050757 (23.04.2009 Gazette 2009/17)**

(54) **METHOD AND INSTRUMENT FOR THE NON-INVASIVE MEASUREMENT OF THE OXYGENATION/SATURATION OF BIOLOGICAL TISSUE**

VERFAHREN UND INSTRUMENT ZUR NICHT-INVASIVEN MESSUNG DER OXYGENIERUNG/SÄTTIGUNG VON BIOLOGISCHEM GEWEBE

PROCÉDÉ ET INSTRUMENT POUR LA MESURE NON INVASIVE DE L'OXYGÉNATION/LA SATURATION DE TISSU BIOLOGIQUE

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **19.10.2007 IT BS20070161**

(43) Date of publication of application:
**04.08.2010 Bulletin 2010/31**

(73) Proprietor: **Nirox S.r.l.**
**25010 Acquafredda (BS) (IT)**

(72) Inventors:
• **BANDERA, Andrea**
**I-25010 Acquafredda (Brescia) (IT)**

• **DONINI, Maurizio**
**I-25010 Acquafredda (Brescia) (IT)**
• **ROVATI, Luigi**
**I-25010 Acquafredda (Brescia) (IT)**

(74) Representative: **Sangiacomo, Fulvia**
**Biesse S.r.l.**
**Via Corfù, 71**
**25124 Brescia (IT)**

(56) References cited:
**EP-A- 0 102 816      US-A- 5 413 100**
**US-A- 5 596 987      US-A- 5 720 284**
**US-A- 5 755 226      US-A1- 2006 200 014**
**US-B2- 6 785 568**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### Field of the Invention

This invention concerns the diagnostic equipment sector and refers in particular to a non-invasive instrument to measure the oxygenation, which is the ration between the concentration of oxygenated and reduced haemoglobin in biological tissue, in particular in the human body.

### State of the art

In general, to evaluate the state of human tissue, such as for example, the brain or a muscle, it is important also to know the level of oxygenation. In this regard, instruments for measuring the oxygenation of tissues are already available.

The important factor is that the examination to be carried out is not invasive. A measuring technique is represented by the NIRS (Near InfraRed Spectroscopy), that consists in illuminating a tissue with a light source with wavelengths in the near infrared spectral range (600-1000nm), in detecting the light diffused by the tissue by means of a receiver and elaborating this data to calculate the concentration of oxygenated haemoglobin, deoxygenated haemoglobin and thus obtaining the oxygenation state of a tissue. This technique is based on the natural transparency of the tissues to light in the NIR region combined with the fact that the tissues contain a series of chromophores, that is substances that absorb the light intensity, of physiological interest with optical absoprtion characteristics that are different from each other. In particular, the chromofores of greater interest are water, lipids, oxygenated haemoglobin and reduced haemoglobin. While some of these are presumed to be constant for a brief period, haemoglobin, both reduced and oxygenated, has concentration strongly related to tissue metabolism. The impinging light penetrates the tissues and undergoes two main processes: diffusion (scattering) and absorption, both depending on the wavelength of the light. In the NIR spectral band the scattering is dominant compared to the absorption, which is mainly due to the haemoglobin.

Oxygenated and reduced haemoglobin have however different absorption spectrums in the NIR which enables the two haemoglobin forms to be measured separately; once the haemoglobin has been quantified the oxygenation level of the tissue can be determined.

The above being given, the choice of the correct algorithm to be used to interpret the data and consequently the realisation and calibration procedures of the instruments, is very important.

The NIRS instruments, depending on the technological approach, differ in the following typologies:

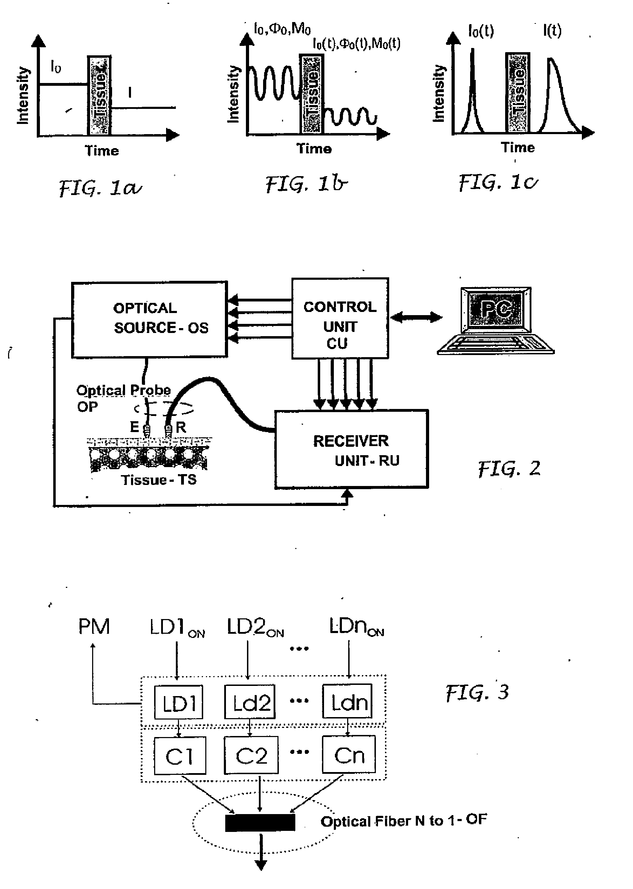

- Continuous intensity (CW): the tissue is illuminated at different wavelengths and/or different source-receiver distances maintaining the power emitted constant (Fig. 1 a);
- Modulated intensity or frequency-domain (FD): the incident light on the tissue is modulated in amplitude at frequencies of between 100-200MHz (Fig. 1 b);
- Time-domain (TD): the tissue is illuminated by impulses having a temporal duration on the order of picoseconds (Fig. 1c).

The TD and FD technological choices, even allowing for the carrying out of a direct measuring of the absorption and scattering coefficients and a quantitative measure of the concentrations of oxygenated haemoglobin, reduced haemoglobin and oxygenation index of the tissue, are characterized by a greater realisation complexity.

The CW instrumentation, on the other hand, does not allow itself the direct measurement of the absorption and scattering coefficient; however, as this approach is the easiest to carry out, in order to achieve quantitative measure different approaches have been brought into being; such as:

- multi-distance systems, which use more distances from the source and the receiver to measure the oxygenation level,
- derivative systems, that carry out second derivatives on the absorption spectrum to eliminate the scattering contribution.

The first approach however, is subject to errors due to the heterogeneous structure of the tissues, whereas with the second approach it is not possible to measure the absolute concentration of oxygenated and reduced haemoglobin.

Also known is the method of the absorption peak of which is based on the fact that the absorption of light travelling into tissue and having certain wavelengths, for example 980 nm, is mainly dominated by water. This method has however been theorized to calculate only the changes of the oxygenated and reduced haemoglobin concentrations and not its quantification.

The document US-A-5 720 284 concerns an apparatus for measuring haemoglobin in which light of various wavelengths emitted from light sources is, however, transmitted through vital tissues and is converted into electric signals to calculate the values of tissue terms and the haemoglobin concentration.

The document US2006/200014 A1 discloses a method for optical measurements of desired parameters of the patient's blood effected, however, creating a state of blood flow cessation within a measuring region, during a time period, and carrying on, in said time period, measurement sessions, each of which includes at least two measurements with different wavelengths of incident light.

The object of this invention is to propose an instrument and a method for the non-invasive quantification of the concentration of oxygenated haemoglobin and deoxygenated haemoglobin; thus providing a measurement of the oxygenation/saturation of biological tissues in a precise manner and at a reduced cost.

A further objective is to propose an instrument particularly, but not only, to measure the oxygenation of a muscle, able to supply a quantitative measurement of the trend of said oxygenation, plus indications regarding the accumulation of lactic acid.

Said objectives and implicit advantages deriving from them are achieved by means of a non-invasive instrument for the measuring of oxygenation/saturation of a biological tissue according to claim 2 and by a method of measuring according to claim 1.

## Brief Description of the Drawings

The invention will however be illustrated more in detail in the description that follows made in reference to the enclosed indicative and not restrictive drawings, in which:

Figs. 1a, b and c are indicative of the technology of the state of the art referred to beforehand;
Fig. 2 is a flow diagram of the measuring instruments according to the invention;
Fig. 3 is a flow diagram of the optics source;
Fig. 4 is a schematic view of the optics probe;
Fig. 5 is a flow diagram of the reception unit;
Fig. 6 is a schematic representation of the measuring cycle;
Fig. 7 is a flow diagram of the control software;
Fig. 8 is an example of a trace as displayed on a PC; and
Fig. 9 shows a data sub-packet.

## Detailed Description of the Invention

According to the invention the theory of the absorption peak of the water was adopted at the study of the oxygenation of a tissue, carried out by an optoelectronic instrument, which, in association with a hardware part and relative piloting and data elaboration software is able to measure the characteristic optical absorption of a material at certain wavelengths belonging to the near infrared spectrum (NIR). From these measurings and in the particular field of application of biological tissue (muscular and cerebral), by means of an opportune elaboration of the data collected, an absolute level of the oxygenated haemoglobin concentration ($HbO_2$) and reduced haemoglobin (Hb), can be obtained that enables the oxygenation/saturation of a tissue to be established and displayed in real-time.

In an example of a realisation such as the one shown in Fig. 2, the instrument is made up of basically of:

an optical source that generates radiation NIR at a continual intensity necessary for measuring;
an optical probe that directs, by means of an optical injection fiber, the light generated by the source onto the tissue and collects, by means of a light guide, the light that exit from the tissue being measured

An optical receiver unit that converts the light coming from the tissue into an electric signal and amplifies it correctly;
a control unit that manages the timing of the system, the analog to digital conversion of the measurement signals and that control the communication with an external personal computer.

The optical source is made up of a preset n number of independent modules, three or more, whose function is to generate an opportune light radiation with wavelength in the NIR spectral range.

Each module comprises a diode laser LD, appropriately controlled by a digital signal Ldi (with i=1.2....n), and an optical coupler C able to couple the optical signal generated into an output optical fiber. The light beams generated by said modules are then conveyed on a sole measuring point by using a N to 1 type fiber optic cable.

The source is also equipped with a system able to measure the optical power generated and to supply a voltage signal, defined as Power Monitor PM, which is proportional to the optical power and that will be acquired by the control unit.

In order to be able to obtain the absolute concentrations of haemoglobin with a continuous wave instrument which in itself would supply only a measurement of the changes in the concentrations, the invention proposes a method based on the water absorption peak method, using the optical absorption of water at a set wavelength, of 980 nm as a reference to calculate the contribution of the scattering at the same wavelength. Given that the scattering coefficient is linearly related with the wavelength of the light and that the coefficients of this relationship are known for many tissues, once the scattering coefficient at 980nm has been measured, it can be calculated for the other wavelengths of the system.

The light intensity that exits the tissue at a given geometric distance between emitter and receiver is a function of the input optical intensity, of the distance between emitter and receiver, of the scattering coefficient and of the absorption coefficient. Once the spectrum of the scattering coefficient has been established, and the other variables being known, it is possible to calculate the absorption coefficient at the other wavelengths. To implement this processing method it is necessary to illuminate the tissue with at least three wavelengths, at least one of which must coincide with a water absorption peak, at 980 nm. This calls for the need to have available an optical source with at least three luminous radiation emitter modules of different wavelengths. In fact, in order to further improve the accuracy of the measurement it is possible to use two or more light source of different wavelengths centered on the same number of water absorption peaks. In this case, for example, the light source module can have at least four modules, two of which with wavelenghts coinciding with the water peaks and the other two with wavelengths not in coincidence with the water absorption peaks.

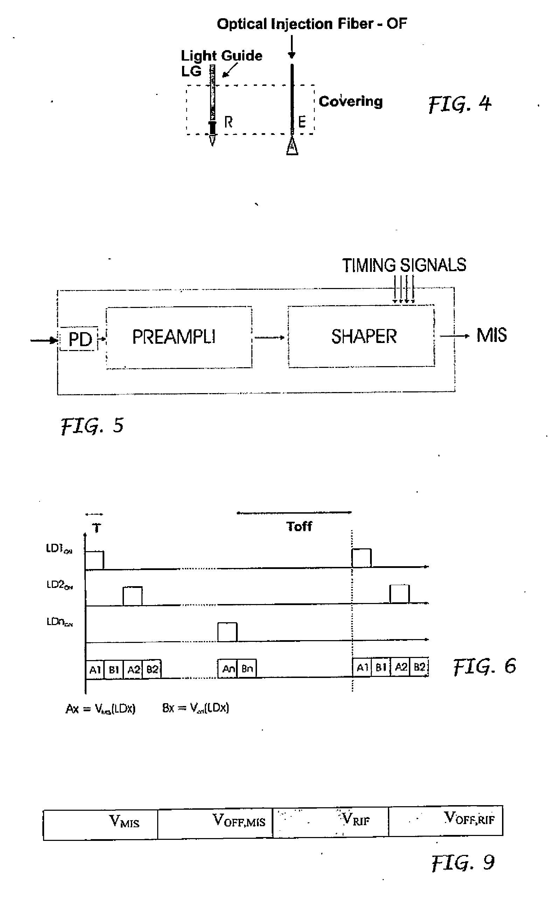

The optical probe -Fig. 4- consists in a covering containing a emitter E and a receiver R to be placed directly in contact with the tissue to be examined, respectively to deliver the light to the tissue and to collect the optically attenuated signal back-scattered from the tissue. Preferably the receiver comprises a liquid light guide because it has greater flexibility characteristics compared to fiber optic bundle and enables the collection area to be widened, thus increasing the sensitivity of the instrument. In order to minimize the dimensions, and in particular the thickness of the external covering a system has been studied to deviate and convey the signal coming from the tissue inside the light guide without having to use bends which are cumbersome and potentially damaging for the light guide.

The receiver unit -Fig. 5- is a high performance electronic sub-system in terms of sensitivity and signal noise ratio able to detect low-level optical signal. It is made up of a photodetector, PD, which can be a photodiode or an avalanche photodiode, APD, which converts the optical signal delivered by the liquid light guide in a current signal proportional to the intensity of the optical radiation. A very low noise preamplifier, PREAMPLI, convert the current into a voltage signal and amplify it.

The voltage output of the preamplifier is then processed by an analog elaboration block, called a Shaper, which opportunely filters the signal and maximizes the signal to noise ratio; the output of this stage is a voltage signal, called MIS, that is proportional to the optical intensity coming from the tissue. To make the instrument easy to use automatic adjustments have been introduced. For this purpose the Shaper block comprises a circuit for the automatic adjustment of the electronic gain based on a digital potentiometer, that allows adapting the sensitivity of the instrument to the measurement of the tissues with different absorption characteristic and a compensation circuit of the offset that enables minimizing the effect of the external light in real time by adding an equal signal to it, but opposite in sign. The final Shaper block was designed considering an ideal rectangular impulse as an input; therefore the optimum shaping circuit is characterized by a response to a rectangular type impulse, feasible with a synchronised integration stage with the signal.

The shaper has a second stage that amplifies the power monitor signal PM coming from the light source and generating a RIF signal proportional to the optical intensity injected into the tissue. The control unit is a digital system based on a microcontroller that manages the timing of the switching on of the lasers and the synchronization of the Shaper. It also takes care of the analogical-digital conversion and sampling of the voltage signals MIS and RIF and their digital transmission to the personal computer where the control software elaborates the data collected to obtain the tissue oxygenation measures. The control unit is therefore the entity that regulates the measuring cycle, that is, all those operations necessary to obtain all the data needed to take a single oxygenation measurement. The duration of the measuring cycle however determines the measuring frequency that is, the number of oxygenation data a second that the system is able to measure. Thanks to the sensitivity characteristics and signal-noise ratio the instrument is able to reach a maximum measuring frequency of 40 Hz.

A single measuring cycle -Fig.6- consists in the sequential switching on of each laser diode that generate the light for a period of time T. During this period of time T the tissue is stimulated by a radiation characterized by a set of wavelength $\lambda$ and by a constant intensity $I_i$ (A); at the same time, the light exiting from the tissue, which has a mitigated intensity $I_o(\lambda)$, is measured by the receiver unit. After time T, the laser is switched off and the receiver unit integrates for the same time T the detected signal, with the aim of measuring the offset that had superimposed over the stimulation signal.

One the switching on of all the lasers has been completed the measuring cycle stops with a $T_{OFF}$ wait period during which the tissue is not stimulated and the data collected is transmitted to the personal computer.

This time is necessary in order to delivery a total optical power in accordance with the standard for a safe use of lasers, in particular the skin maximum permissible exposure.

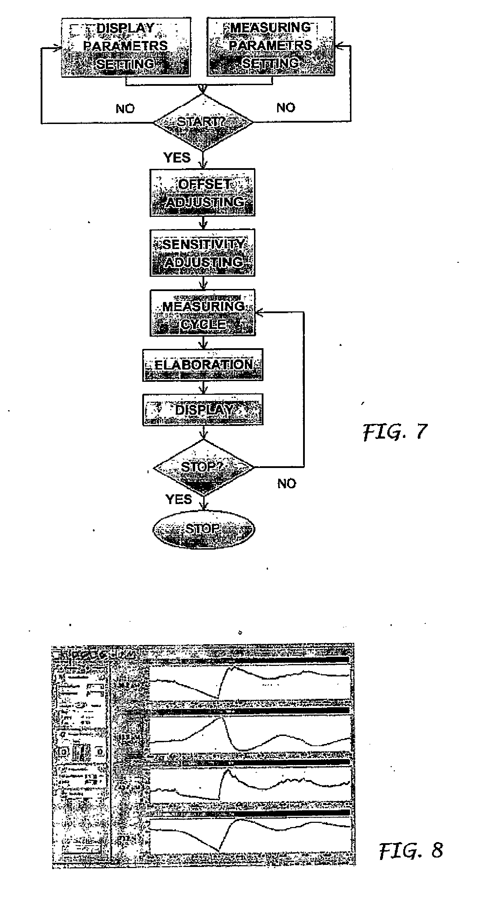

As shown in the flow diagram of the control software in Fig. 7, once started, the control software initializes an interface with the PC to enable the user to set the measuring and display parameters, waiting for the START signal. When it arrives, the software automatically adjusts the offset and sensitivity parameters of the instrument interpreting the signals arriving in certain conditions.

On terminating this self-adjustment phase the instrument is ready to carry out the real time measurement repeating the measuring cycle until it receives a STOP signal. At the same time the software receives the data supplied by the instrument, it elaborates them as described below and displays the traces detected on the PC monitor. Once the STOP signal has been received the instrument stops the measuring cycle and waits for a new START command.

In case of muscle tissue oxygenation measurements the user can select the muscle (upper or lower muscles) under investigation; this allows the software to correctly select the parameters that describe the relationship of the scattering coefficient with wavelength because these parameters change for different tissues.

To set up the measuring parameters, choose the type of muscle, start and interrupt the measuring cycle and display the data in real time, the instrument has been equipped with a simple and intuitive user interface, that displays the instantaneous reading and temporary evolution of the muscular oxygenation state.

The principal functions are:

- adjustment of the display parameters
- choice of muscle examined
- evaluating and zooming of traces
- saving and printing of the measurement
- assisted calculation of the main parameters that can be indirectly measured with nir spectroscopy, in rest condition or during exercise (oxygen consumption, blood flow, deoxygenation rate, recovery time, inflection point, etc.).

At each measuring cycle the control software receives a data pack made up of n sub-packs (where n is the wavelength number constituting the source). Each sub-pack is made up of two pairs of voltage levels regarding the measurement channel that is, the electric signal proportional to the received optical power, and to the reference channel, that is the electric signal proportional to the delivered optical power. Each pair of voltage levels represents the measured signal and the offset superimposed on it as in Fig. 9.

The processing of the data is made up of two main phases:

- Optical absorption calculation: in this first step the voltage signal are processed to obtain the value of the delivered optical power level and the value of the optical power back-scattered from the tissue
- Absolute concentrations calculation: in this step the optical absorption data are processed by means of an inversion algorithm that calculate the absolute concentrations of oxygenated haemoglobin c[HbO2] and reduced haemoglobin c[HHb], from which total haemoglobin c[tHb]=c[HbO2]+c[HHb] and the oxygenation index of the tissue TOI=100*c[HbO2]/c[tHb] are obtained.

The optical absorption, expressed in optical density OD (Optical Density), for each wavelength A, is calculated using the equation:

$$OD(\dot{\lambda}) = \log\left(\frac{I_i}{I_o}\right)$$

where $I_i$ represents the optical intensity (expressed in mW) incident on the tissue, whereas $I_o$ represents the back-scattered optical intensity (expressed in mW).

The incident intensity on the tissue is given by the equation:

$$I_i(\lambda) = k_1(\lambda) * k_2(\lambda) * k_3(\lambda) * (V_{RIF}(\lambda) - V_{OFF,RIF}(\lambda))$$

where $k_1$ takes into consideration the sensitivity of the laser source, $k_2$ of the attenuation of the fibre n to 1, $k_3$ of the electronic amplification.

The retro diffused intensity from the tissue is given by the equation:

$$I_o(\lambda) = k_{m1}(\lambda) * k_{m2}(\lambda) * (V_{MIS}(\lambda) - V_{OFF,MIS})(\lambda))$$

where $k_{m1}$ takes into consideration the sensitivity of the photodetector module, $k_{m2}$ of the electronic amplification.

The constants $k_i$ and $k_{mi}$ are obtained by the characterization of each single functional block of the instrument.

The optical absorption obtained in this way is then corrected by a calibration straight line, obtained when the instrument is assembled on a series of reference samples, by means of the following relation:

$$OD_m(\lambda) = A * OD + B$$

where A and B are the angular coefficient and the intercept specific of each wavelength obtained by the calibration procedure and $OD_m$ is the corrected optical absorption level.

This correction curve make possible the taking into account of possible changes in the optical probe, for example should be replaced or suffered of slight damage, that does not jeopardize its functioning, but which somehow modifies the power level transmitted and/or received, without having to characterize again every single functional block.

As regards to the calculation of the absolute concentrations, this passage represents the central crux of the elaboration, which is carried out by the water absorption peak method.

This process is based on the use of one or more wavelengths for which the hypothesis is valid that in correspondence with such spectral tone there are one or more chromophores that have a known concentration and whose contribution to the total absorption coefficient is dominant.

The theory that describes the propagation of the NIR photons inside the scattering medium, such as the muscle tissue, is based on the diffusion equation. Such an equation can be solved analytically in the case of simple geometry, such as for example a semi-infinite and homogeneous medium, assuming that the photons cover a random path dominated by the high level of the scattering coefficient.

The analytic solution $R_T$ of the diffusion equation for a semi-infinite and homogeneous medium assuming that the light source emits continuous intensity is as follows:

$$R_T(d) = \frac{\left[z_o^+ \times \frac{(1 + \mu_{eff} \times \rho^+)}{(\rho^+)^3} \times \exp(-\mu_{eff} \times \rho^+)\right] - \left[z_o^- \times \frac{(1 + \mu_{eff} \times \rho^-)}{(\rho^-)^3} \times \exp(-\mu_{eff} \times \rho^-)\right]}{4\pi}$$

$$z_o^+ = \frac{1}{\mu'_s} \qquad z_o^- = -(z_o^+ + 2z_e)$$

$$\rho^+ = \sqrt{d^2 + z_0^{+2}} \qquad \rho^- = \sqrt{d^2 + z_0^{-2}}$$

$$\mu_{eff} = \sqrt{3\mu_a \mu'_s}$$

$$z_e = 6A_p\mu'_s$$

$$A_p = 3.0846 - 6.5312n + 8.3579n^2 - 5.0828n^3 + 1.1714n^4 \quad \text{[Eq. 1]}$$

where n is the relative refraction index (that is the ratio between the internal and external refraction index), d is the source-receiver distance, $\mu_a$ is the absorption coefficient of the medium, $\mu'_s$ is the reduced scattering coefficient of the medium.

This equation [Eq. 1] gives the theoretical reflectance level depending on the various parameters indicated; given that the absorption and scattering coefficients are multiplied between them, it is not possible to separate the contribution; consequently the typical continuous wave instrumentation was only able to measure changes in the chromophores of interest.

According to the invention, to quantify the absolute concentration of oxygenated and reduced haemoglobin, the total absorption coefficient at a wavelength of 980 nm, calculated as follows, must be taken into consideration:

$$\mu_a(980nm) = \alpha_{HbO2}(980nm) \times c[HbO2] + \alpha_{HHb}(980nm) \times c[HHb] + \alpha_{H2O}(980nm) \times c[H2O]$$

The water absorption peak method is based on the following assumptions:

1) With a typical concentrations of c[HbO2]=60uM, c[HHb]=20uM and c[H2O]=80% the water contribution to the absorption coefficient at 980nm is predominant;
2) The scattering coefficient is linearly dependent with the wavelength.

Furthermore to be able to compare the diffusion equation [Eq. 1] with an experimental measurement, the area and the collection angle of the receiver must be taken into account. In fact this equation has been obtained assuming that the receiver is punctiform and has a 180° collection angle. In an experimental setup the receiver is characterized by a discrete collection area and a specific collection angle. To measure this difference a specific experimental procedure has been adopted to accurately evaluate a calibration constant; this procedure exploits a series of measurements on calibration phantoms with known optical properties.

The function input parameters are:

1) OD($\lambda_1, \lambda_2, ... , \lambda_{n-1}, \lambda_n$): optical absorption measured at n wavelengths
2) $d_{SR}$: geometrical distance between source and receiver
3) $\alpha_{HbO2}(\lambda_1, \lambda_2, ... , \lambda_n)$: extinction coefficients specific for oxygenated haemoglobin at the wavelengths required
4) $\alpha_{HHb}(\lambda_1, \lambda_2, ... , \lambda_n)$: extinction coefficients specific for reduced haemoglobin at the wavelengths required
5) $\alpha_{H2O}(\lambda_1, \lambda_2, ... , \lambda_n)$: extinction coefficients specific for water at the wavelengths required
6) Type of tissue: depending on the part it belongs to (cerebral, upper or lower muscles) the slope of the relation between the scattering coefficient and wavelength is selected.
7) csi: a parameter that takes into consideration the collection area and angle, that differs according to the type of receiver
8) $C_{H2O}$: water concentration at the tissular level (default level 80%)

The mathematical steps to obtain the absolute concentrations are:

1) For each wavelength at disposal $R_T$ is calculated as 10^(-OD)
2) Each $R_T$ level is multiplied by the constant csi
3) The $\mu_a$ (980nm) level is calculated starting from the specific extinction coefficient and from the tissue water concentration (should not be supplied the software use the default one for 80%)
4) The equation [Eq. 1] is inverted with an iterative numerical method by inserting the $\mu_a$ level calculated in the previous step, the level $R_T$(980nm) measured experimentally and therefore deducing the scattering coefficient at 980nm
5) The scattering coefficient varies linearly with the wavelength and the slope of this relation depends on the type of tissue investigated. By using the coefficient scattering level of 980 nm also the scattering coefficient of the other wavelengths can be calculated by using the equation:

$$\mu'_s(\lambda_i) = \mu'_s(980nm) + a \times (980 - \lambda_i)$$

6) For each wavelength, except for 980nm, taking into account the reduced scattering coefficient calculated in the previous step, the $R_T$ level measured experimentally and by iterative numerical inversion of the equation [Eq. 1] the absorption coefficient can be calculated.
7) Knowing the absorption coefficients at each wavelength, the concentration of the chromophores can be found, according to the Lambert-Beer law, with a least-square inversion of the following matrix:

$$\begin{cases} \mu_a(\lambda_1) = \alpha_{HbO2}(\lambda_1) \times c[HbO2] + \alpha_{HHb}(\lambda_1) \times c[HHb] \\ \mu_a(\lambda_2) = \alpha_{HbO2}(\lambda_2) \times c[HbO2] + \alpha_{HHb}(\lambda_2) \times c[HHb] \\ \qquad\qquad \mathbf{M} \\ \mu_a(\lambda_n) = \alpha_{HbO2}(\lambda_n) \times c[HbO2] + \alpha_{HHb}(\lambda_n) \times c[HHb] \end{cases}$$

8) Knowing the absolute concentrations of oxygenated haemoglobin c[HbO2] and reduced haemoglobin c[HHb], the total haemoglobin concentration c[tHb]=c[HbO2]+c[HHb] and the oxygenation index of the tissue TOI=100*c[HbO2]/c[tHb] can be found.

This data is then sent to the control unit that generates a graph like the one in Fig. 8, to be displayed on a PC, to supply the continuous progress of the state of oxygenation of the tissue being examined.

This instrument was studied in particular but not exclusively, for sport medicine application and for the study of muscle tissue oxygenation. To supply data which can be used for the functional evaluation and setting up of a sporting activity a correlation between the onset of accumulation of blood lactic acid and the shape of the oxygenation trace was studied and found. Specifically, it has been established that when the oxygenation level decreases according to a curve with a slope greater than a set limit level, established through experiments, a contemporary accumulation of lactic acid can be observed. The instrument can be equipped with acoustic and/or visible signalling means to warn the user when the oxygenation curve starts to drop with a slope nearing the limit level, so as to calibrate the sporting activity. The advantage is that such limit level is not theoretical and does not depend in any way on the age or training level of the user, but it is absolutely objective and therefore the instrument gives a real indication and does not require any setting, other than in relation to the type of muscle being examined.

The instrument can be made using a configuration like the one described previously, to be used for example in a medical center, in a gymnasium or connected to gymnastic equipment such as a treadmill, a bicycle ergometer or some other equipment, for more precise measurements, in which optical source, receiver unit and control unit are positioned inside a piece of equipment connected to the probe to be applied to muscle under test and to a PC to view in real time the oxygenation progress, the signalling means and the various displays for initial adjustments.

Also programmed is a "portable" version for direct use during an activity, where the optical source and receiver unit are both placed inside the probe to be applied to the muscle being examined, whereas the control unit is placed in a piece of equipment with reduced dimensions, that communicates, by means of a connection with or without wires, with the receiver unit and which contains means for setting and displaying the initial parameters and an acoustic and/or visible signaller. This piece of equipment can, secondly, be connected to a PC to "download" the graph of the sporting activity and display it by means of an appropriate software.

## Claims

1. A method for non-invasive measuring the state of the oxygenation/saturation of a human or animal tissue (TS) having a given water concentration, such as the brain or a muscle, comprising, following the choice of the tissue to be analysed, the stages:

   - providing an instrument for the non-invasive measurement of the oxygenation/saturation of biological tissue comprising at least one optical source (OS) generating continuous intensity luminous radiations, an optical probe (OP) comprising an emitter (E) to convey the light radiations generated by the at least one optical source (OS) onto a part of the tissue (TS) and a receiver (R) to collect the radiations backscattered from the tissue, a receiver unit (RU) used to convert and amplify an optical signal coming from said receiver (R) into an output electrical signal (MIS), wherein the receiver unit (RU) is an electronic sub-system for detecting low-level optical signals and comprises a photodetector (PD) for the conversion of optical radiations coming from the tissue to the receiver (R) into a current signal proportional to them, a preamplifier (PREAMPLI) to convert and amplify the current to a voltage signal, and an analogical elaboration block (SHAPER) adapted to filter and maximize the signal to noise ratio and to supply an output signal proportional to the optical intensity back scattered from the tissue and comprising a gain adjustment circuit for the automatic adjustment of the gain to adapt the instrument to measure tissues with different absorption levels, and a compensation circuit adapted to minimize the external light effect in real time by adding a signal equal to it, but opposite in sign, and an integration stage synchronised with the signal itself an electronic control unit (CU) adapted to manage timings. measured signals and an interface to an external personal computer (PC) through a software for the elaboration of the collected electric signals to obtain absolute levels of the oxygenation of the tissue;
- setting up and adjusting the instrument according to the type of tissue:
- placing the emitter (E) and receiver (R) of the optical probe (OP) in contact with the tissue (TS);
- starting the measuring cycle by using an appropriate signal;
- the emitter (E) emitting luminous radiations at a given optical intensity and at least at three different wavelengths in the near infrared spectrum, where at least one is 980 nm, correspondent to a water absorption peak for a localized illumination of the tissue;
- the gain adjustment circuit automatically adjusting of the electronic gain by means of a digital potentiometer, so as to adapt the sensitivity of the instrument to the absorption level of the tissue examined, andcompensating the offset by means of said compensation circuit, so as to minimize the external light intensity by adding an equal signal to it, but opposite in sign,
- the receiver (R) collecting the luminous radiations backscattered from the tissue at a set distance from the illuminated zone;
- transforming the backscattered radiation detected into an electric signal by means of said photodetector (PD) and amplify the electric signal by means of said low noise preamplifier (PREAMPLI);the electronic control unit (CU):
- calculating the optical absorption, expressed in optical density, for each wavelength depending on the optical intensity incident on the tissue and

backscattering optical intensity;

- calculating the reflectance for each wavelength of the radiations emitted;
- multiplying each reflectance by a constant depending on the type of the receiver;
- calculating the absorption coefficient at the 980 nm wavelength, according to the water specific absorption coefficient and concentration;
- calculating the reflectance level according to the photon migration theory in tissues and in dependence of their scattering inside the tissue;
- inverting the procedure of calculating the reflectance based on the absorption coefficient and the reflectance measured at the wavelength 980 nm to retrieve the diffusion coefficient (scattering) in the tissue at said wavelength;
- using the calculated scattering coefficient to deduce the scattering coefficients of the other wavelengths as well;
- inverting the calculation procedure of the reflectance on the basis of the calculated scattering coefficient and the reflectance measured for each wavelength to retrieve the absorption coefficient for each wavelength;
- calculating the absolute concentrations of oxygenated and reduced haemoglobin, according to the Lambert-Beer law using the absorption coefficients previously calculated;
- calculating the absolute concentration of total haemoglobin as a sum of the haemoglobin oxygen and reduced haemoglobin concentrations and the oxygenation index of the tissue as the ratio of the oxygenated haemoglobin concentration over the total haemoglobin concentration;
- memorizing and possibly displaying in real time on the personal computer (PC) monitor the calculated concentrations.

2. An instrument for the non-invasive measurement of the state of the oxygenation/ saturation of biological tissue both human and animal, such as the brain or a muscle, by the method according to claim 1, comprising at least one optical source (OS) generating continuous intensity luminous radiations, an optical probe (OP) comprising an emitter (E) to convey the light radiations generated by the at least one optical source (OS) onto a part of the tissue and a receiver (R) to collect the radiations backscattered from the tissue, a receiver unit (RU) used to convert and amplify the optical signal coming from said receiver (R) into an output electrical signal (MIS), wherein the receiver unit (RU) is an electronic sub-system for detecting low-level optical signals and comprises a photodetector (PD) for the conversion of optical radiations coming from the tissue to the receiver (R) into a current signal proportional to them, a preamplifier (PREAMPLI) to convert and amplify the current to a voltage signal, and an analogical elaboration block (SHAPER) adapted to filter and maximize the signal to noise ratio and to supply an output signal proportional to the optical intensity back scattered from the tissue and comprising a gain adjustment circuit for the automatic adjustment of the gain to adapt the instrument to measure tissues with different absorption levels, and a compensation circuit adapted to minimize the external light effect in real time by adding a signal equal to it, but opposite in sign, and an integration stage synchronised with the signal itself, an electronic control unit (CU) adapted to manage timings, measured signals and an interface to an external personal computer (PC) through a software for the elaboration of the electric signals collected to obtain absolute levels of the oxygenation of the tissue to display in real time the obtained absolute levels, wherein the optical source (OS) is made up of at least three independent optical modules (LD1 +C1, LD2+C2, ...,LDn+Cn) for the emission of respective different luminous radiations with wavelengths belonging to the near infrared spectrum, where at least one of said radiations is correspondent to a water absorption peak at 980 nm, and where the emitter (E) and receiver (R) are at a distance set according to the level of the power of the optical source.

3. An instrument according to claim 2, in which the optical source (OS) comprises four independent optical modules, two of which being for emission of luminous radiations with wavelengths in correspondence with respective water absorption peaks and two of which being for an emission of respective light radiation with wavelengths not in correspondence with the water absorption peaks.

4. An instrument according to claims 2 or 3, in which each optical module of the optical source (OS) comprises a laser diode (LD), controlled by a digital signal, an optical coupler (C) to couple the optical signals generated into an optical fiber (OF), to deliver all signals exiting from each module to a single point, and a system to measure the optical power generated to supply a proportional output voltage signal to the electronic control unit (CU).

5. An instrument according to any one of claims 2 to 4, in which the receiver (R) includes a liquid light guide (LG) to increase the collection area and the sensitivity of the instrument.

6. An instrument according to any of claims 2 to 5, in which optical emitter and receiver are on the same support and the optical source and the receiver unit are placed inside the optical probe, the receiver unit communicating with the electronic control unit by means of a wireless or wire connection.

7. An instrument according to any one of claims 2 to 6, for the use, in combination with display devices and/or acoustic and/or visual alarm devices indicative of extreme levels of the oxygenation progress, in monitoring the oxygenation progress even in real time during a sporting activity.


**Patentansprüche**

1. Verfahren zur nichtinvasiven Messung des Zustandes der Sauerstoffanreicherung/-sättigung eines menschlichen oder tierischen Gewebes (TS) mit einer gegebenen Wasserkonzentration, wie das Gehirn oder einen Muskel, umfassend folgende Schritte, nach der Auswahl des zu analysierenden Gewebes:

   - Bereitstellen eines Gerätes zur nichtinvasiven Messung der Sauerstoffanreicherung/-sättigung von biologischem Gewebe, umfassend mindestens eine optische Quelle (OS) zur Erzeugung von Lichtstrahlungen mit konstanter Intensität, eine optische Sonde (OP) mit einem Sender (E) zum Leiten der von der mindestens einen optischen Quelle (OS) erzeugten Lichtstrahlung auf einen Teil des Gewebes (TS) und einem Empfänger (R) zum Aufnehmen der vom Gewebe zurückgestreuten Strahlungen, eine Empfängereinheit (RU), die zur Umwandlung und Verstärkung eines aus dem besagten Empfänger (R) kommenden optischen Signals in ein elektrisches Ausgangssignal (MIS) dient, wobei die Empfängereinheit (RU) ein elektronisches Untersystem zur Detektion von optischen Niederpegel-Signalen ist, umfassend einen Photodetektor (PD) zur Umwandlung der aus dem Gewebe bis zum Empfänger (R) verlaufenden optischen Strahlungen in ein dazu proportionales Stromsignal, einen Vorverstärker (PREAMPLI) zur Umwandlung und Verstärkung des Stromsignals in ein Spannungssignal und einen analogen Verarbeitungsblock (SHAPER), der zum Filtern und zur Maximierung des Signal-Rausch-Verhältnisses sowie zum Bereitstellen eines Ausgangssignals proportional zur vom Gewebe zurückgestreuten optischen Intensität geeignet ist, weiter umfassend eine Verstärkungseinstellschaltung zur automatischen Einstellung der Verstärkung, um das Gerät an die Messung von Geweben mit verschiedenen Absorptionsgraden anzupassen, und eine Kompensationsschaltung, die zur Echtzeit-Minimierung der Fremdlichteinwirkung durch Addieren eines dazu gleichwertigen, aber mit entgegengesetztem Vorzeichen versehenen Signals geeignet ist, und eine mit dem Signal selbst synchronisierte Integrationsstufe, eine elektronische Steuereinheit (CU), die zur Verwaltung der zeitlichen Abläufe, der gemessenen Signale und einer Schnittstelle für einen externen Personal Computer (PC) durch eine Software zur Verarbeitung der aufgenommenen elektrischen Signale geeignet ist, um den absoluten Sauerstoffanreicherungspegel des Gewebes zu erhalten;
   - Einrichten und Einstellen des Gerätes nach dem Gewebetyp;
   - Verbinden des Senders (E) und des Empfängers (R) der optischen Sonde (OP) mit dem Gewebe (TS);
   - Starten des Messzyklus durch Benutzung eines passenden Signals;
   - Emittieren durch den Sender (E) einer Lichtstrahlung mit gegebener optischer Intensität und mit mindestens drei verschiedenen Wellenlängen im nahen Infrarotspektrum, wobei wenigstens eine davon 980 nm beträgt, entsprechend des Absorptionsspitzenwertes des Wassers zur örtlichen Beleuchtung des Gewebes;
- Automatisches Regeln durch die Verstärkungseinstellschaltung der elektronischen Verstärkung anhand eines digitalen Potentiometers, um die Empfindlichkeit des Gerätes an den Absorptionspegel des analysierten Gewebes anzupassen, und
- Ausgleichen des Offset anhand der besagten Kompensationsschaltung, um die Fremdlichtintensität durch Addieren eines dazu gleichwertigen, aber mit entgegengesetztem Vorzeichen versehenen Signals zu minimieren,
- Aufnehmen der vom Gewebe zurückgestreuten Strahlungen durch den Empfänger (R) bei einem eingestellten Abstand bezüglich der beleuchteten Zone,
- Umwandeln der ermittelten zurückgestreuten Strahlung in ein elektrisches Signal mittels des besagten Photodetektors (PD) und Verstärken des elektrischen Signals mittels des besagten rauscharmen Vorverstärker (PREAMPLI);

wobei die elektronische Steuereinheit (CU):

- die als optische Dichte ausgedrückte optische Absorption für jede Wellenlänge in Abhängigkeit der auf das Gewebe einfallenden optischen Intensität und der zurückgestreuten optischen Intensität berechnet;
- das Reflexionsvermögen für jede Wellenlänge der emittierten Strahlungen berechnet;
- jedes Reflexionsvermögen mit einer Konstante in Abhängigkeit des Empfängertyps multipliziert;
- den Absorptionskoeffizient bei 980 nm Wellenlänge in Abhängigkeit des bzw. der spezifischen Absorptions-Koeffizient und Absorptions-Konzentration des Wassers berechnet;
- den Reflexionsgrad nach der Photonenmigration-Theorie in Geweben und in Abhängigkeit der Streuung innerhalb des Gewebes berechnet;
- das Verfahren zum Berechnen des Reflexionsvermögens auf der Basis des Absorptionskoeffizienten und des bei einer Wellenlänge von 980 nm gemessenen Reflexionsvermögens umkehrt, um den Diffusionskoeffizienten (Streuung) im Gewebe bei der besagten Wellenlänge wieder aufzufinden;
- den berechneten Streuungskoeffizienten zum Ableiten auch der Streuungskoeffizienten der weiteren Wellenlängen benutzt;
- das Verfahren zum Berechnen des Reflexionsvermögens auf der Basis des berechneten Streuungskoeffizienten und des für jede Wellenlänge gemessenen Reflexionsvermögens umkehrt, um den Absorptionskoeffizienten für jede Wellenlänge wieder aufzufinden;
- die absoluten Konzentrationen von oxygeniertem und reduziertem Hämoglobin nach dem Lambert-Beer-Gesetz bei Benutzung des zuvor berechneten Absorptionskoeffizienten berechnet;
- die absolute Konzentration des Gesamt-Hämoglobins als Summe der Konzentrationen des Hämoglobinsauerstoffes und des reduzierten Hämoglobins und den Sauerstoffindex des Gewebes als Verhältnis der Konzentration des oxigenierten Hämoglobins zum Gesamt-Hämoglobin berechnet;
- die berechneten Konzentrationen abspeichert und möglicherweise in Echtzeit auf dem Bildschirm des Personal Computer (PC) anzeigt.

2. Gerät zur nichtinvasiven Messung der Sauerstoffanreicherung/-sättigung von biologischem Gewebe, sowohl eines menschlichen als auch eines tierischen Gewebes, wie des Gehirns oder eines Muskels, mit dem Verfahren nach Anspruch 1, umfassend mindestens eine optische Quelle (OS) zur Erzeugung von Lichtstrahlungen mit konstanter Intensität, eine optische Sonde (OP) mit einem Sender (E) zum Leiten der von der mindestens einen optischen Quelle (OS) erzeugten Lichtstrahlung auf einen Teil des Gewebes und einen Empfänger (R) zum Aufnehmen der vom Gewebe zurückgestreuten Strahlungen, eine Empfängereinheit (RU), die zur Umwandlung und Verstärkung eines aus dem besagten Empfänger (R) kommenden optischen Signals in ein elektrisches Ausgangssignal (MIS) dient, wobei die Empfängereinheit (RU) ein elektronisches Untersystem zur Detektion von optischen Niederpegel-Signalen ist und einen Photodetektor (PD) zur Umwandlung der aus dem Gewebe bis zum Empfänger (R) verlaufenden optischen Strahlungen in ein dazu proportionales Stromsignal umfasst, einen Vorverstärker (PREAMPLI) zur Umwandlung und Verstärkung des Stromsignals in ein Spannungssignal und einen analogen Verarbeitungsblock (SHAPER), der zum Filtern und zur Maximierung des Signal-Rausch-Verhältnisses sowie zum Bereitstellen eines Ausgangssignals proportional zur vom Gewebe zurückgestreuten optischen Intensität geeignet ist, weiter umfassend eine Verstärkungseinstellschaltung zur automatischen Einstellung der Verstärkung, um das Gerät an die Messung von Geweben mit verschiedenen Absorptionsgraden anzupassen, und eine Kompensationsschaltung, die zur Echtzeit-Minimierung der Fremdlichteinwirkung durch Addieren eines dazu gleichwertigen, aber mit entgegengesetztem Vorzeichen versehenen Signals geeignet ist, und eine mit dem Signal selbst synchronisierte Integrationsstufe, eine elektronische Steuereinheit (CU), die zur Verwaltung der zeitlichen Abläufe, der gemessenen Signale und einer Schnittstelle für einen externen Personal Computer (PC) durch eine Software zur Verarbeitung der aufgenommenen elektrischen Signale geeignet ist, um absolute Sauerstoffanreicherungspegel des Gewebes zu erhalten, zur Echtzcit-Anzeige der erhaltenen Absolutpegel, wobei die optische Quelle (OS) aus mindestens drei selbstständigen optischen Modulen (LD1+C1, LD2+C2, ...,LDn+Cn) zur Emission von entsprechenden verschiedenen Lichtstrahlungen mit zu dem nahen Infrarotspektrum gehörenden Wellenlängen, wobei mindestens eine der besagten Strahlungen der Wasserabsorptionsspitze bei 980 nm entspricht und wobei der Sender (E) und der Empfänger (R) mit einem Abstand zueinander angeordnet sind, der in Abhängigkeit vom Leistungspegel der optischen Quelle eingestellt ist.

3. Gerät nach Anspruch 2, wobei die optische Quelle (OS) vier unabhängige optische Module aufweist, von denen zwei zur Emission von Lichtstrahlungen mit Wellenlängen bei den entsprechenden Wasserabsorptionsspitzen und zwei zur Emission von Lichtstrahlungen mit Wellenlängen, die den Wasserabsorptionsspitzen nicht entsprechen, eingerichtet sind.

4. Gerät nach Anspruch 2 oder 3, wobei jedes optische Modul der optischen Quelle (OS) eine von einem Digitalsignal gesteuerte Laserdiode (LD), einen optischen Koppler (C) zur Kopplung des in einer optischen Faser (OF) erzeugten optischen Signals und zur Abgabe aller von jedem Modul austretenden Signale in einem einzelnen Punkt und ein System zur Messung der optischen Leistung, die zur Versorgung einer elektronischen Steuereinheit (CU) mit einem proportionalen Ausgangs-Spannungssignal erzeugt wird, umfasst.

5. Gerät nach einem der Ansprüche 2 bis 4, wobei der Empfänger (R) einen Flüssigkeitslichtleiter (LG) umfasst, um die Aufnahmezone und die Empfindlichkeit des Gerätes zu erhöhen.

6. Gerät nach einem der Ansprüche 2 bis 5, wobei der optische Sender und Empfänger auf demselben Träger angeordnet sind und die optische Quelle und die Empfängereinheit innerhalb der optischen Sonde platziert sind, wobei die Empfängereinheit mit der Steuereinheit mittels Drahtverbindung oder drahtloser Verbindung kommuniziert.

7. Gerät nach einem der Ansprüche 2 bis 6, wobei es zur Benutzung bei der Überwachung des Ablaufes der Sauerstoffanreicherung, sogar in Echtzeit während einer sportlichen Aktivität, in Kombination mit Bildschirmeinrichtungen und/oder akustischen und/oder visuellen Alarmvorrichtungen, welche die Extrempegel des Sauerstoffanreicherungsablaufes anzeigen, vorgesehen ist.

## Revendications

1. Procédé de mesure non-invasive de l'état de l'oxygénation/saturation d'un tissu humain ou animal (TS) ayant une concentration d'eau donnée, par exemple le cerveau ou un muscle, comprenant, après le choix du tissu à analyser, les étapes de:

   - fournir un instrument pour la mesure non invasive de l'oxygénation/saturation d'un tissu biologique comprenant au moins une source optique (OS) produisant radiations lumineuses avec une intensité continue, une sonde optique (OP) comprenant un émetteur (E) pour adresser les radiations lumineuses générées par l'au moins une source optique (OS) sur une partie du tissu (TS) et un récepteur (R) pour recueillir les radiations rétrodiffusées à partir du tissu, une unité de réception (RU) utilisée pour convertir et amplifier un signal optique provenant dudit récepteur (R) en un signal électrique de sortie (MIS), où l'unité de réception (RU) est un sous-système électronique pour détecter des signaux optiques de faible intensité et comprend un photodétecteur (PD) pour convertir des radiations optiques provenant du tissu et arrivant au récepteur (R) en un signal de courant proportionnel à eux, un préamplificateur (PREAMPLI) pour convertir le courant en un signal de tension et pour l'amplifier, et un bloc d'élaboration analogique (SHAPER) adapté pour filtrer et maximiser le rapport signal-bruit et pour fournir un signal de sortie proportionnel à l'intensité optique rétrodiffusée à partir du tissu et comprenant un circuit de réglage de gain pour le réglage automatique du gain pour adapter l'instrument à mesurer les tissus présentant des niveaux d'absorption différentes, et un circuit de compensation adapté pour minimiser l'effet de la lumière externe en temps réel par l'addition d'un signal égal à ceci, mais opposé en signe, et un étage d'intégration synchronisé avec le signal lui-même, une unité de commande électronique (CU) adaptée pour gérer les temporisations, les signaux mesurés et une interface avec un ordinateur personnel externe (PC) par l'intermédiaire d'un software pour l'élaboration des signaux électriques recueillis pour obtenir des niveaux absolus de l'oxygénation des tissus;
   - préparer et régler l'instrument selon le type de tissu;
   - placer l'émetteur (E) et le récepteur (R) de la sonde optique (OP) en contact avec le tissu (TS);
   - commencer le cycle de mesure en utilisant un signal approprié;

- l'émetteur (E) en émettant des radiations lumineuses avec une intensité optique donnée et au moins à trois longueurs d'ondes différentes dans le spectre du proche infrarouge, où au moins une est de 980 nm, correspondant à un pic d'absorption d'eau pour une illumination localisée du tissu;

- le circuit de réglage de gain en réglant automatiquement le gain électronique au moyen d'un potentiomètre numérique, de manière à adapter la sensibilité de l'instrument au niveau d'absorption du tissu examiné, et en compensant le décalage au moyen dudit circuit de compensation, de manière à minimiser l'intensité de la lumière extérieure en ajoutant un signal égal à elle, mais de signe opposé,

- le récepteur (R) en recueillant des radiations lumineuses rétrodiffusées à partir du tissu à une distance établie de la zone éclairée;

- transformer la radiation rétro diffusée détectée en un signal électrique par l'intermédiaire dudit photodétecteur (PD) et amplifier le signal électrique par l'intermédiaire dudit préamplificateur à faible niveau de bruit (PREAMPLI); l'unité de commande électronique (CU):

- en calculant le facteur d'absorption optique, exprimé en densité optique, pour chaque longueur d'onde en fonction de l'intensité optique sur le tissu et

en rétro diffusant la densité optique;

- en calculant le facteur de réflexion pour chaque longueur d'onde des radiations émises;

- en multipliant chaque facteur de réflexion par une constante basée sur le type de récepteur;

- en calculant le coefficient d'absorption au niveau de la longueur d'onde de 980 nm, selon le coefficient d'absorption spécifique et la concentration de l'eau;

- en calculant le niveau de réflexion en fonction de la théorie de la migration de photons dans les tissus et en fonction de leur diffusion à l'intérieur du tissu;

- en intervertissant la procédure de calcul du facteur de réflexion sur la base du coefficient d'absorption et du facteur de réflexion mesuré au niveau de la longueur d'onde de 980 nm pour récupérer le coefficient de diffusion (dispersion) dans le tissu au niveau de ladite longueur d'onde;

- en utilisant les coefficients de diffusion calculés pour déduire les coefficients de diffusion même des autres longueurs d'onde;

- en intervertissant la procédure de calcul du facteur de réflexion sur la base du coefficient de diffusion calculé et du facteur de réflexion mesuré pour chaque longueur d'onde pour récupérer le coefficient d'absorption pour chaque longueur d'onde;

- en calculant les concentrations absolues d'hémoglobine oxygénée et réduite, conformément à la loi de Beer-Lambert en utilisant les coefficients d'absorption calculés précédemment;

- en calculant la concentration absolue de l'hémoglobine totale sous forme de somme de l'oxygène de l'hémoglobine et des concentrations réduites d'hémoglobine et l'indice d'oxygénation du tissu sous forme du rapport de la concentration d'hémoglobine oxygénée sur la concentration de l'hémoglobine totale;

- en mémorisant et éventuellement en affichant en temps réel les concentrations calculées sur l'ordinateur personnel (PC).

2. Instrument de mesure non-invasive de l'état de l'oxygénation/saturation d'un tissu humain ou bien animal, par exemple le cerveau ou un muscle, par le procédé selon la revendication 1, comprenant au moins une source optique (OS) produisant radiations lumineuses avec une intensité continue, une sonde optique (OP) comprenant un émetteur (E) pour adresser les radiations lumineuses générées par l'au moins une source optique (OS) sur une partie du tissu et un récepteur (R) pour recueillir les radiations rétrodiffusées à partir du tissu, une unité de réception (RU) utilisée pour convertir et amplifier le signal optique provenant dudit récepteur (R) en un signal électrique de sortie (MIS), dans lequel l'unité de réception (RU) est un sous-système électronique pour détecter des signaux optiques de faible intensité et comprend un photodétecteur (PD) pour convertir des radiations optiques provenant du tissu et arrivant au récepteur (R) en un signal de courant proportionnel à eux, un préamplificateur (PREAMPLI) pour convertir le courant en un signal de tension et pour l'amplifier, et un bloc d'élaboration analogique (SHAPER) adapté pour filtrer et maximiser le rapport signal-bruit et pour fournir un signal de sortie proportionnel à l'intensité optique rétrodiffusée à partir du tissu et comprenant un circuit de réglage de gain pour le réglage automatique du gain pour adapter l'instrument à mesurer les tissus présentant des niveaux d'absorption différentes, et un circuit de compensation adapté pour minimiser l'effet de la lumière externe en temps réel par l'addition d'un signal égal à ceci, mais opposé en signe, et un étage d'intégration synchronisé avec le signal lui-même, une unité de commande électronique (CU) adaptée pour gérer les temporisations, les signaux mesurés et une interface avec un ordinateur personnel externe (PC) par l'intermédiaire d'un software pour l'élaboration des signaux électriques recueillis pour obtenir des niveaux absolus de l'oxygénation des tissus pour afficher en temps réel les signaux électriques recueillis, dans lequel la source optique (OS) est constituée d'au moins trois modules optiques indépendants (LD1 + C1, LD2 + C2,

..., LDn + Cn) pour l'émission de différentes radiations lumineuses respectives avec longueurs d'onde appartenant au spectre du proche infrarouge, où au moins l'une desdites radiations est correspondant à un pic d'absorption de l'eau de 980 nm, et où la distance de l'émetteur (E) et de le récepteur (R) est fixée en fonction du niveau de la puissance de la source optique.

3. Instrument selon la revendication 2, dans lequel la source optique (OS) comprend quatre modules optiques indépendants, deux d'entre eux destinés à l'émission de radiations lumineuses avec longueurs d'onde en correspondance avec des pics d'absorption de l'eau respectifs et deux d'entre eux destinés à une émission de radiations lumineuses respectives avec des longueurs d'onde qui ne sont pas en correspondance avec les pics d'absorption de l'eau.

4. Instrument selon la revendication 2 ou 3, dans lequel chaque module optique de la source optique (OS) comprend une diode laser (LD), commandé par un signal numérique, un coupleur optique (C) pour coupler les signaux optiques générés dans une fibre optique (OF), pour emmener tous les signaux sortant de chaque module à un seul point, et un système pour mesurer la puissance optique générée pour fournir à l'unité de commande électronique (CU) un signal proportionnel de tension de sortie.

5. Instrument selon l'une quelconque des revendications 2 à 4, dans lequel le récepteur (R) comprend un guide de lumière liquide (LG) pour augmenter la zone de recueil et la sensibilité de l'instrument.

6. Instrument selon l'une quelconque des revendications 2 à 5, dans lequel l'émetteur et le récepteur optique sont sur le même support et la source optique et l'unité de réception sont placées à l'intérieur de la sonde optique, l'unité de réception communiquant avec l'unité de commande électronique au moyen d'une connexion sans fil ou d'une connexion filaire.

7. Instrument selon l'une quelconque des revendications 2 à 6 pour l'utilisation, en combinaison avec des dispositifs d'affichage et/ou des dispositifs d'alarme sonores et/ou visuels indiquant des niveaux extrêmes de l'avancement de l'oxygénation, dans la surveillance de l'avancement de l'oxygénation pendant une activité sportive, même en temps réel.

**FIG. 1a**

**FIG. 1b**

**FIG. 1c**

**FIG. 2**

**FIG. 3**

**Optical Injection Fiber - OF**

**Light Guide**
**LG**

Covering    *FIG. 4*

R    E

TIMING SIGNALS

PD → PREAMPLI → SHAPER → MIS

*FIG. 5*

$Ax = V_{MIS}(LDx)$    $Bx = V_{OFF}(LDx)$

*FIG. 6*

| $V_{MIS}$ | $V_{OFF,MIS}$ | $V_{RIF}$ | $V_{OFF,RIF}$ |
|-----------|---------------|-----------|---------------|

*FIG. 9*

FIG. 7

FIG. 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5720284 A
- US 2006200014 A1